# EUROPEAN PATENT APPLICATION

(11) **EP 2 540 303 A1**
(43) Date of publication of application: **02.01.2013**
(21) Application number: 12174209.2
(22) Date of filing: 28.06.2012
(51) Int. Cl.: A61K 33/00, A61K 33/06, A61K 33/14, A61K 33/30, A61K 33/34, A61P 17/00, A61P 17/02

(54) **Divalent cation containing compositions and methods for treating and/or preventing enzymatic irritation**

(30) Priority: 28.06.2011 US 201113170264; 28.06.2011 US 201113170277
(71) Applicant: Johnson & Johnson Consumer Companies Inc., Skillman, NJ 08558 (US)
(72) Inventor: Lin, Connie B, Belle Mead, NJ New Jersey 08502 (US); Gunn, Euen Thomas Graham Ekman, Hopewell, NJ New Jersey 08525 (US); Hu, Ya-Ping, Highland Park, NJ New Jersey 08904 (US); Kulkarni, Neema, Randolph, NJ New Jersey 07869 (US); Mack, Mary Catherine, Summit, NJ 07901 (US)
(74) Representative: Carpmaels & Ransford

(57) **Abstract**

Compositions and methods for treating and/or preventing conditions such as diaper rash and atopic dermatitis are disclosed. The compositions and methods are particularly useful in the treatment and prevention of diaper rash and diaper dermatitis caused by the prolonged contact of human skin with body waste. The methods employ the topical application of a trypsin-inhibiting agent to the area in need of such treatment, or the area where prevention is desired. The trypsin-inhibiting agent is preferably a divalent cation.

## Description

### FIELD OF THE INVENTION

The invention relates to the use of divalent cations to protect the skin of a human. More particularly, the invention relates to the use of divalent cations in a skin care composition that can be topically applied to protect skin from irritants, and in particular protease enzymes, and more in particular trypsin. The present invention is useful in the prevention and treatment of diaper rash and the mitigation of atopic dermatitis.

### BACKGROUND OF THE INVENTION

The skin is a natural barrier to the penetration of foreign substances. The stratum corneum is the superficial cornified layer of the skin that provides a barrier to water evaporation and reduces the permeation of undesirable molecules from the external environment. The stratum corneum consists of dead cells called corneocytes, which are embedded in a lipid-rich matrix of fatty-acids, ceramides, and cholesterols. This structure of corneocytes embedded in lipids is thought to provide many of the barrier properties of the skin. Substances deposited on the skin must traverse this structure to gain access to the underlying viable layers of the skin. Skin inflammation occurs when substances which are irritating to the skin are able to penetrate this barrier and initiate the secretion of inflammatory mediators once they contact the skin cells in the viable epidermis and dermis layers. As the skin barrier is compromised, skin is subject to inflammatory events from percutaneous absorption of irritants through the stratum corneum.

Skin barrier function can be compromised by a variety of insults that cause inflammation. One such insult caused by exposure to body fluids and waste results in what is commonly known as diaper rash. Bodily fluids and wastes may contain skin irritants in the form of enzymes such as proteases, ureases, and lipases. Enzymes found in feces cleave the stratum corneum proteins and lipids causing the breakdown of the natural barrier of the skin and release of irritating agents such as free fatty acids. Bacterial ureases on the skin convert the urea in urine to ammonia resulting in an alkaline pH on the skin. Prolonged exposure of the skin to these enzymes is thought to be a major cause of skin damage that leads to dermatitis and subsequent skin breakdown.

A number of approaches are known for protecting the skin against the action of proteases and subsequent skin breakdown, including skin protectant formulations, and anti-inflammatory compositions. Many of the skin protectant formulations commercially available may not provide adequate protection against skin irritants. Many of these formulations consist of softening creams and hydrating compounds to replenish the moisture content of the skin. However, these formulations do not block the irritants present in urine, feces, or blood such as, e.g., the proteolytic enzymes present in feces. Furthermore these compositions often consist of petrolatum, lanolin or greasy compounds that can rub off onto garments and decrease the absorbency of the garment.

Another method of treating skin irritation includes the use of anti-inflammatory compounds. However, this method does not protect the skin from coming in contact with an irritant, therefore, damage to the skin still occurs. The anti-inflammatory substance mitigates the inflammatory response but it does not prevent the skin damage that elicits the inflammatory event in the first place.

Hahn of Cosmederm Technologies discloses examples where divalent cations at amounts of about 60 mM or greater inhibit skin irritation caused by chemical irritants or environmental conditions. See, e.g., U.S. Patent No. 6,455,076; W09619182; W09619181; U.S. Patent No. 5,958,436 and U.S. Patent No. 5,804,203.

U.S. Published Application No. 20040102429 to Modak et al. discloses anti-irritant compositions that contain two or more water-soluble organic salts of zinc at concentrations between *0.1% and 2%.

U.S. Patent No. 5,965,610 to Columbia University discloses zinc gluconate containing topical compositions which have an anti-irritant effect on skin.

U.S. Patent No. 4,477,439 to D'Alelio et al. discloses the use of sulfates or phosphates of barium, calcium, strontium or zinc to reduce irritated or excoriated areas surrounding the stoma of ostomy patients.

Baby skin is especially sensitive to environmental and dietary conditions. Frequent exposure to feces and urine can promote bacterial growth that is known to cause skin irritation and infection. Consequent to this exposure, excessive amounts of lipase and proteolytic enzymes, and their metabolic products such as ammonia and fatty acids, are released and cause skin irritation and allergic skin rashes.

The skin problems discussed herein generally extend to humans at the early and late portions of life. That is, infants, as well as the elderly, are often subject to these skin problems.

One of the most common conditions of infant skin is diaper rash, also known as diaper dermatitis. The primary contributors to the development of diaper rash have long been thought to be agents and enzymatic activities within infant urine and feces. Atherton, A review of the pathophysiology, prevention and treatment of irritant diaper dermatitis, Curr. Med. Res. Opin., 20:645-649 (2004); Wolf et al., Diaper dermatitis, Clin. Dermatol., 18:657-660 (2000).

However, merely keeping the area clean and dry does not protect the irritated skin from the irritation associated with the by-products of infant urine and feces. U.S. Patent No. 5,436,007 to Abbott Laboratories discloses a composition that contains a linear polydimethylsiloxane polymer, a non-ionic emulsifier consisting of polyoxyethylene sorbitan fatty acid esters, sorbitan fatty acid esters, polyoxyethylene alcohols, or polyoxyethylene fatty ethers, aloe vera, an alkoxylated ether/ester, sodium citrate, citric acid, a blend of propylene glycol, diazolidinyl urea, methyl paraben and propyl paraben, for use in the protection and treatment of diaper rash.

Great Britain Patent No. 1,357,731 to Lake discloses a powder composition that can be incorporated into a hydrophobic ointment. A buffer system is provided to buffer the composition at a pH of from 5.5 to 7.5.

U.S. Patent No. 4,556,560 to the Proctor & Gamble Company discloses examples of the use of lipase inhibiting agents at amounts of about 10 mM or greater, such as water soluble metallic salts including zinc chloride, in the treatment of diaper rash.

U.S. Patent No. 4,816,254 to Moss discloses a composition for treating skin irritations such as diaper rash that contains zinc oxide, boric acid, karaya gum, Peruvian balsam, cod liver oil, solvent and a pharmaceutical carrier.

U.S. Patent No. 4,911,932 to Johnson and Johnson Consumer Products, Inc. discloses a skin care composition that may be used for diaper rash that contains zinc oxide and miconazole nitrate.

U.S. Published Application No. 20050175719 to Sun et al. discloses the use of a procyanidin to prevent or treat skin rash.

U.S. Published Application No. 20070237834 to Bioderm Research discloses the use of divalent metal complexes of zeolites, such as zinc zeolite, to treat diaper rash.

Many skin care products aiming to reduce the symptoms of or to relieve diaper rash contain zinc oxide, which forms a protective barrier. Common products include diaper rash and sunscreen compounds. Although the formulations are often effective at providing a barrier, they are often greasy. Zinc oxide is insoluble and appears to be inert, that is, it appears not to react to mitigate an inflammatory response or to react with irritants to negate the damage from urine or feces. Zinc oxide alone does not have significant protease inhibition capability.

It is now understood that the initial stages of some types of diaper rash are the result of skin irritation caused by contact with digestive enzymes present in infant feces, particularly trypsin, chymotrypsin and elastase. Among these enzymes are proteolytic and lipolytic enzymes produced in the gastrointestinal tract to digest food. In infants, the feces tend to be watery and contain, among other materials such as bacteria, some amounts of digestive enzymes. These enzymes, if they remain in contact with the skin for any appreciable period of time, have been found to cause an irritation that is uncomfortable and can predispose the skin to infection by microorganisms.

Proteases occur naturally in all organisms. These enzymes are involved in a multitude of physiological reactions. Proteases can either break specific peptide bonds (limited proteolysis), depending on the amino acid sequence of a protein, or break down a complete peptide to amino acids (unlimited proteolysis).

Proteases are involved in digesting long protein chains into short fragments, splitting the peptide bonds that link amino acid residues. Some of them can detach the terminal amino acids from the protein chain (exopeptidases, such as aminopeptidases, carboxypeptidase A); others attack internal peptide bonds of a protein (endopeptidases, such as trypsin, chymotrypsin, pepsin, papain, and elastase).

Proteases are divided into four major groups according to the character of their catalytic active site and conditions of action: serine proteinases, cysteine (thiol) proteinases, aspartic proteinases, and metalloproteinases. Attachment of a protease to a certain group depends on the structure of catalytic site and the amino acid (as one of the constituents) essential for its activity.

Proteases are used throughout an organism for various metabolic processes. For example, acid proteases secreted into the stomach (such as pepsin) and serine proteases present in duodenum (trypsin and chymotrypsin) enable us to digest the protein in food.

Trypsin is secreted into the duodenum, where it acts to hydrolyse peptides into their smaller building-blocks, namely amino acids (these peptides are the result of the enzyme pepsin's breaking down the proteins in the stomach). This enables the uptake of protein in the food because peptides (though smaller than proteins) are too big to be absorbed through the lining of the ileum. Trypsin catalyses the hydrolysis of peptide bonds.

The enzymatic mechanism is similar to that of other serine proteases. These enzymes contain a catalytic triad consisting of histidine-57, aspartate-102, and serine-195. These three residues form a charge relay that serves to make the active site serine nucleophilic. This is achieved by modifying the electrostatic environment of the serine. The enzymatic reaction that trypsins catalyze is thermodynamically favorable but requires significant activation energy (it is "kinetically unfavorable"). In addition, trypsin contains an "oxyanion hole" formed by the backbone amide hydrogen atoms of Gly-193 and Ser-195, which serves to stabilize the developing negative charge on the carbonyl oxygen atom of the cleaved amides.

The aspartate residue (Asp 189) located in the catalytic pocket (S1) of trypsins is responsible for attracting and stabilizing positively-charged lysine and/or arginine, and is, thus, responsible for the specificity of the enzyme. This means that trypsin predominantly cleaves proteins at the carboxyl side (or "C-terminal side") of the amino acids lysine and arginine, except when either is bound to a c-terminal proline. Trypsins are considered endopeptidases, i.e., the cleavage occurs within the polypeptide chain rather than at the terminal amino acids located at the ends of polypeptides.

Green et al., The effects of divalent cations on trypsin, J. Biol. Chem., 204:379-390 (1953), discloses that metal ions such as Hg++, Cu++, Ag+ and to a lesser extent Zn++ inhibited the esterase activity of trypsin at low concentrations, i.e., around 0.001 M.

Toyota et al., Synthesis and evaluation of guanidine-containing Schiff base copper (II), zinc (II) and iron (II) chelates as trypsin inhibitors, Chem. Pharm. Bull., 51(6): 625-629 (2003), discloses the study of Schiff base metal chelates inhibitory activity against trypsin.

Jane et al., A novel approach to serine protease inhibition: kinetic characterization of inhibitors whose potencies and selectivities are dramatically enhanced by zinc (II), Biochemistry, 39: 4792-4800 (2001), discloses that zinc (II) potentiates the inhibitory interaction between low-molecular weight chelating inhibitors and trypsin.

Proteases in the skin are essential to epidermal permeability barrier homeostasis. In addition to their direct proteolytic effects, certain proteases signal to cells by activating protease-activated receptors (PARs), the G-protein-coupled receptors. The expression of functional PAR-2 on human skin and its role in inflammation, pruritus, and skin barrier homeostasis have been demonstrated. Atopic dermatitis (AD) is a multifactorial inflammatory skin disease characterized by genetic barrier defects and allergic inflammation, which is sustained by gene-environmental interactions. Recent studies have revealed aberrant expression and activation of serine proteases and PAR-2 in the lesional skin of AD patients. The imbalance between proteases and protease inhibitors associated with genetic defects in the protease/protease inhibitor encoding genes, increase in skin surface pH, and exposure to proteolytically active allergens contribute to this aberrant protease/PAR-2 signaling in AD. The increased protease activity in AD leads to abnormal desquamation, degradation of lipid-processing enzymes and antimicrobial peptides, and activation of primary cytokines, thereby leading to permeability barrier dysfunction, inflammation, and defects in the antimicrobial barrier. Moreover, up-regulated proteases stimulate PAR-2 in lesional skin of AD and lead to the production of cytokines and chemokines involved in inflammation and immune responses, itching sensation, and sustained epidermal barrier perturbation with easier allergen penetration. In addition, PAR-2 is an important sensor for exogenous danger molecules, such as exogenous proteases from various allergens, and plays an important role in AD pathogenesis. Lee et al. disclose that protease activity or PAR-2 may be a future target for therapeutic intervention for the treatment of AD. See Lee et al., Protease and Protease-Activated Receptor-2 Signaling in the Pathogenesis of Atopic Dermatitis, Yonsei Med J. 2010 November 1; 51(6): 808-822.

What is needed, therefore, is a skin care composition that can be applied directly to the skin to block the activity of enzymes such as trypsin found in bodily fluids, feces or inflamed skin. This need is provided by the present invention. The invention has particular relevance to diaper rash and atopic dermatitis.

### SUMMARY OF THE INVENTION

The present invention describes compositions and methods for treating and/or preventing inflammatory dermatoses such as enzymatic irritation and in particular enzymatic dermatitis, such as diaper rash and atopic dermatitis, via the topical administration of divalent cations.

The present invention also describes compositions and methods for treating and/or preventing inflammatory dermatoses such as enzymatic irritation, and in particular enzymatic dermatitis, such as diaper rash and atopic dermatitis, via the topical administration of divalent cation/anion pairs, otherwise referred to herein as divalent cation salts.

The present invention also describes compositions and methods for treating and/or preventing inflammatory dermatoses such as enzymatic irritation, and in particular enzymatic dermatitis, such as diaper rash and atopic dermatitis, via the topical administration of a specified divalent cation, i.e., magnesium, and talc. The magnesium may be present in the composition in its divalent cation salt form.

The present invention also describes compositions and methods for treating and/or preventing inflammatory dermatoses such as enzymatic irritation, and in particular enzymatic dermatitis, such as diaper rash and atopic dermatitis, via the topical administration of zinc oxide and acid so as to release a specified divalent cation, i.e., zinc.

Other features and advantages of the present invention will be apparent from the detailed description of the invention and from the claims.

### BRIEF DESCRIPTION OF THE FIGURES

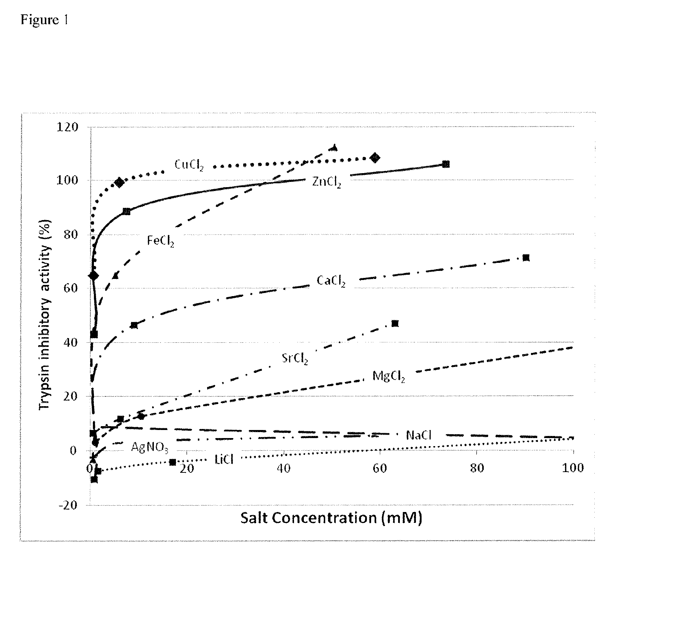

Figure 1 is a graph showing the effect of various cations on the inhibition of trypsin.

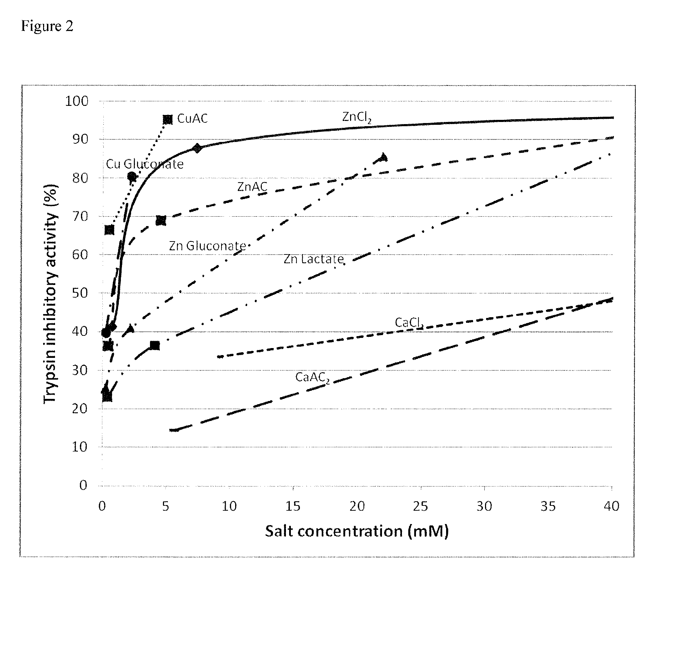

Figure 2 is a graph showing the effect of various salt forms of divalent cations on the inhibition of trypsin at various concentrations in mM.

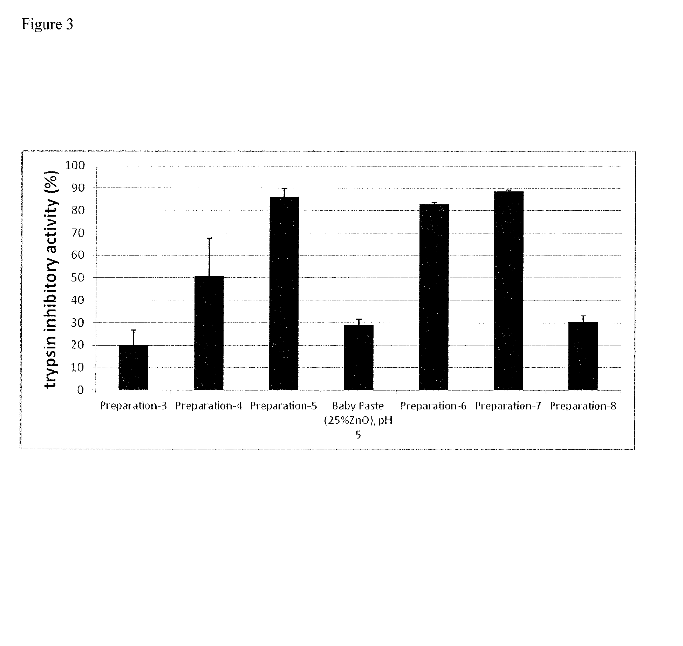

Figure 3 is a graph showing (1) the effect of various amounts of citric acid in a zinc oxide containing formulation on the inhibition of trypsin and (2) a comparison of the effect of citric acid in a zinc oxide containing formulation on the inhibition of trypsin with the effect of Baby Paste 5.0, a commercially available product from Mendelson Pharmaceuticals LLC, Mountainside, NJ, on the inhibition of trypsin.

### DETAILED DESCRIPTION OF THE INVENTION

Unless defined otherwise, all technical and scientific terms used herein have the same meaning as commonly understood by one of ordinary skill in the art to which the invention belongs. Also, all publications, patent applications, patents, and other references mentioned herein are incorporated by reference. As used herein, all percentages are by weight unless otherwise specified.

As used herein, "trypsin inhibitory activity" means the ability to inhibit the activity of the protease, trypsin, as measured by the assay set forth below in the examples. The divalent cations used in the present invention preferably have a trypsin inhibitory activity of at least about 15%. For example, the divalent cations used in the present invention have a trypsin inhibitory activity of at least about 25%, and preferably at least about 50%, or at least about 60%, or at least about 70%, or at least about 80%, or at least about 90%.

As used herein, "prevent" or "preventing" means to proactively stop the development of enzymatic skin irritation, or to halt or slow down the progression of such irritation, or to reduce the risk of developing such irritation.

As used herein, "treat" or "treating" is meant to comfort the skin near and/or at the location of enzymatic skin irritation, and if possible to induce a regression in such irritation. As used herein, "regression" is meant to reduce the amount and/or severity of topical enzymatic skin irritation symptoms, such as, e.g., irritation, redness, blisters, discomfort, excoriation, and the like.

As used herein, "topical application" means directly laying on or spreading on outer skin using, e.g., the hands, an applicator such as a wipe, puff, roller, or spray, or via a substrate such as a diaper.

As used herein, "affected area" is meant the area of skin that is presently exhibiting any level of skin rash or enzymatic dermatitis, or the area that will be in prolonged contact with urine or feces containing such dermatitis-causing enzymes. This area also includes the area immediately proximate to the described area. It is the area at which treatment and/or prevention is desired.

As used herein, "topical carrier" means one or more compatible solid or liquid filler diluents that are suitable for topical administration to a mammal. Examples of topical carriers include, but are not limited to, water, waxes, oils, emollients, emulsifiers, thickening agents, gelling agents, and mixtures thereof.

The topical compositions of the present invention comprise a safe and effective amount of a divalent cation. Suitably, the composition contains, based upon the total amount of topical composition, from about 0.001 % to about 50% divalent cation. For example, the composition contains from about 0.001% to about 30% divalent cation. Preferably, the composition contains from about 0.01 % to about 10% divalent cation. More preferably, the composition contains from about 0.1 % to about 5.0% divalent cation. Most preferably, the composition contains from about 0.1 % to about 1.0% divalent cation.

The composition may contain, based upon the total amount of topical composition, from about 0.001% to about 50% divalent cation salt. Suitably, the composition may contain from about 0.001% to about 30% divalent cation salt. Preferably, the composition may contain from about 0.01 % to about 10% divalent cation salt. More preferably, the composition may contain from about 0.1 % to about 5.0% divalent cation salt. Most preferably, the composition contains from about 0.1 % to about 1.0% divalent cation salt.

The composition may contain from about 0.1 mM to about 5000 mM divalent cation salt. Suitably, the composition contain from about 0.1 mM to about 3000 mM divalent cation salt. Preferably, the composition contains from about 1.0 mM to about 1000 mM divalent cation salt. More preferably the composition contains from about 1.0 mM to about 500 mM divalent cation salt. Most preferably, the composition contains from about 1.0 mM to about 500 mM divalent cation salt.

The present invention discloses the use of divalent cations in compositions for the treatment of diaper dermatitis (diaper rash).

The compositions useful in the present invention involve formulations suitable for topical application to skin. The composition may contain an acceptable topical carrier in an amount, based upon the total weight of the composition, from about 50% to about 99.99%, e.g., from about 80% to about 95%.

The composition may be made into a wide variety of product types that include but are not limited to lotions, creams, gels, sticks, sprays, ointments, cleansing liquid washes and solid bars, pastes, powders, mousses, wipes, patches, wound dressing and adhesive bandages, hydrogels, and films. These product types may comprise several types of acceptable topical carriers including, but not limited to solutions, emulsions (e.g., microemulsions and nanoemulsions), gels, solids and liposomes. The following are non-limitative examples of such carriers. Other carriers can be formulated by those of ordinary skill in the art.

The topical compositions useful in the present invention can be formulated as anhydrous products. As used herein, "anhydrous" shall mean that the compositions contain less than about 10 %, e.g., less than about 5% or less than about 1% water.

Alternatively, the topical compositions useful in the present invention can be formulated as solutions. Solutions typically include water (e.g., from about 50% to about 99.99% or from about 90% to about 99% of water).

Topical compositions useful in the subject invention may be formulated as a solution comprising an emollient. Such compositions may contain from about 2% to about 50% of an emollient(s). As used herein, "emollients" refer to materials used for the prevention or relief of dryness, as well as for the protection of the skin. A wide variety of suitable emollients are known and may be used herein. Sagarin, Cosmetics, Science and Technology, 2nd Edition, Vol. l, pp. 32-43 (1972) and the International Cosmetic Ingredient Dictionary and Handbook, eds. Wenninger and McEwen, pp.1656-61, 1626, and 1654-55 (The Cosmetic, Toiletry, and Fragrance Assoc., Washington, D.C., 7.sup.th Edition, 1997) (hereinafter "CI Handbook") contain numerous examples of suitable materials.

A lotion can be made from such a solution. Lotions typically comprise from about 1% to about 20% (e.g., from about 5% to about 10%) of an emollient(s) and from about 50% to about 90% (e.g., from about 60% to about 80%) of water.

Another type of product that may be formulated from a solution is a cream. A cream typically comprises from about 5% to about 50% (e.g., from about 10% to about 20%) of an emollient(s) and from about 45% to about 85% (e.g., from about 50% to about 75%) of water.

Yet another type of product that may be formulated from a solution is an ointment. An ointment may comprise a simple base of animal or vegetable oils or semi-solid hydrocarbons. An ointment may comprise from about 2% to about 10% of an emollient(s) plus from about 0.1 % to about 2% of a thickening agent(s). A more complete disclosure of thickening agents or viscosity increasing agents useful herein can be found in Sagarin, Cosmetics, Science and Technology, 2nd Edition, Vol. l, pp. 72-73 (1972) and the ICI Handbook pp. 1693-1697.

The topical compositions useful in the present invention can also be formulated as emulsions. If the carrier is an emulsion, from about 1% to about 10% (e.g., from about 2% to about 5%) of the carrier comprises an emulsifier(s). Emulsifiers may be nonionic, anionic or cationic. Suitable emulsifiers are disclosed in, e.g., U.S. Patent No. 3,755,560, U.S. Patent No. 4,421,769, McCutcheon's Detergents and Emulsifiers, North American Edition, pp. 317-324 (1986), and the ICI Handbook, pp.1673-1686.

Lotions and creams can be formulated as emulsions. Typically such lotions comprise from 0.5% to about 5% of an emulsifier(s). Such creams would typically comprise from about 1 % to about 20% (e.g., from about 5% to about 10%) of an emollient(s); from about 20% to about 80% (e.g., from 30% to about 70%) of water; and from about 1% to about 10% (e.g., from about 2% to about 5%) of an emulsifier(s).

Single emulsion skin care preparations, such as lotions and creams, of the oil-in-water type and water-in-oil type are well-known in the cosmetic art and are useful in the subject invention. Multiphase emulsion compositions, such as the water-in-oil-in-water type, as disclosed in U.S. Patents Nos. 4,254,105 and 4,960,764, are also useful in the subject invention. In general, such single or multiphase emulsions contain water, emollients, and emulsifiers as essential ingredients.

The topical compositions of this invention can also be formulated as a gel (e.g., an aqueous gel using a suitable gelling agent(s)). Suitable gelling agents for aqueous gels include, but are not limited to, natural gums, acrylic acid and acrylate polymers and copolymers, and cellulose derivatives (e.g., hydroxymethyl cellulose and hydroxypropyl cellulose). Suitable gelling agents for oils (such as mineral oil) include, but are not limited to, hydrogenated butylene/ethylene/styrene copolymer and hydrogenated ethylene/propylene/styrene copolymer. Such gels typically comprises between about 0.1 % and 5%, by weight, of such gelling agents.

The topical compositions of this invention can also be combined with particulates such as clay, silica and starch such as cornstarch, and the like, and optional flow agents such as tricalcium phosphate, in order to form dusting powders. Examples of such powder components and methods for making powder compositions may be found in, e.g., U.S. Patents Nos. 4,568,539 and 4,485,092.

Liposomal formulations are also useful compositions of the subject invention. Examples of liposomes are unilamellar, multilamellar, and paucilamellar liposomes, which may or may not contain phospholipids. Such compositions can be prepared by first combining hesperetin with a phospholipid, such as dipalmitoylphosphatidyl choline, cholesterol and water according to the method described in Mezei & Gulasekharam, "Liposomes--A Selective Drug Delivery System for the Topical Route of Administration; Gel Dosage Form", Journal of Pharmaceutics and Pharmacology, Vol. 34 (1982), pp. 473-474, or a modification thereof. Epidermal lipids of suitable composition for forming liposomes may be substituted for the phospholipid. The liposome preparation may then incorporated into one of the above carriers (e.g., a gel or an oil-in-water emulsion) in order to produce the liposomal formulation. Other compositions and uses of topically applied liposomes are described in Mezei, M., "Liposomes as a Skin Drug Delivery System", Topics in Pharmaceutical Sciences (D. D. Breimer and P. Speiser, eds.,), Elsevier Science Publishers B. V., New York, N.Y., 1985, pp. 345-358, PCT Patent Application No. WO96/31194 and U.S. Patent No. 5,260,065.

The topical compositions useful in the subject invention may contain, in addition to the aforementioned components, a wide variety of additional oil-soluble materials and/or water-soluble materials conventionally used in compositions for use on skin at their art-established levels.

The composition may also contain one more of the following: antifungals such as ketoconazole, miconazole, elubiol, allantoin, calamine, silicon-based organic polymers such as, but not limited to, polymerized siloxane such as dimethicone, kaolin, petrolatum, white petrolatum, cod liver oil, lanolin, mineral oil, talc, topical starch, and any other agent suitable for use in the treatment and/or prevention of diaper rash.

The composition may contain one or more of the following agents in an amount, based upon the total weight of the composition, from about 0.5% to about 2% of allantoin, from about 1% to about 25% calamine, from about 1 % to about 30% dimethicone, from about 4% to about 20% kaolin, from about 30% to less than about 100% petrolatum, from about 30% to less than about 100% of white petrolatum, from about 5% to about 14% of cod liver oil, e.g., such that the amount of cod liver oil does not exceed 10,000 USP units of vitamin A and 400 USP units of cholecalciferol (where USP is the United States Pharmacopeia Unit, known in the art), about 10% to about 16% of lanolin, from about 50% to less than about 100% mineral oil, and from about 10% to about 87% topical starch. Examples of vitamins include, but are not limited to, vitamin A, vitamin Bs such as vitamin B3, vitamin B5, and vitamin B12, vitamin C, vitamin K, and vitamin E and derivatives thereof.

The present invention discloses a composition comprising; (i) a divalent cation, wherein (ii) said composition is for the treatment of conditions such as diaper dermatitis. Said composition can include an acceptable carrier or base. The divalent cation is selected from, but not limited to, e.g., copper (II), zinc, iron (II), calcium, strontium, magnesium, tin (II), nickel (II), manganese (II), and cadmium (II).

The present invention discloses a composition comprising; (i) a divalent cation/anion pair (otherwise known as a divalent cation salt), wherein (ii) said composition is for the treatment of conditions such as diaper dermatitis. Said composition can include an acceptable carrier or base. The divalent cation/anion pair is selected from, but is not limited to, the divalent cations listed above, i.e., copper (II), zinc, iron (II), calcium, strontium, magnesium, tin (II), nickel (II), manganese (II), and cadmium (II), in combination with anions selected from, e.g., various carboxylic acids selected from, but not limited to, acetic acid, ascorbic acid, aspartic acid, benzoic acid, boric acid, carbonic acid, citric acid, folic acid, gluconic acid, glucuronic acid, glutamic acid, glycyrrhizic acid, glyoxylic acid, hydrochloric acid, hyaluronic acid, lactic acid, lanolic acid, malic acid, niacin, polyacrylic acid, rosamarinic acid, salicylic acid, sorbic acid and tartaric acid. Examples of divalent cation/anion pairs include, but are not limited to, e.g., copper (II) chloride, copper (II) acetate, copper (II) gluconate, copper (II) lactate, zinc chloride, zinc acetate, zinc gluconate, zinc lactate, zinc carbonate, zinc borate, zinc aspartate, zinc pyrrolidone carboxylic acid, zinc citrate, iron (II) chloride, iron (II) acetate, iron (II) gluconate, iron (II) lactate, calcium chloride, calcium acetate, calcium gluconate, calcium lactate, strontium chloride, strontium acetate, strontium gluconate, strontium lactate, magnesium chloride, magnesium acetate, magnesium gluconate and magnesium lactate.

The present invention discloses a composition comprising; (i) a specified divalent cation, i.e., magnesium, in combination with talc, wherein (ii) said composition is for the treatment of conditions such as diaper dermatitis. Said composition can include an acceptable carrier or base. The composition may contain from about 45% to less than about 100% talc.

The present invention discloses a composition comprising; (i) zinc oxide and an acid, that when combined releases a specified divalent cation, i.e., zinc, wherein (ii) said composition is for the treatment of conditions such as diaper dermatitis. Any acid that would result in the release of zinc cation when reacted with zinc oxide can be used. Such acids include, but are not limited to, e.g., citric acid, acetic acid, salicyclic acid, carbonic acid, clucuronic acid, gluconic acid, lactic acid and malic acid. Preferably, zinc oxide is present in an amount selected from the group consisting of from about 1% to about 40%, from about 1% to about 25%, from about 1% to about 20%, from about 1% to about 15%, from about 5% to about 13%, about 1.0 %, about 10%, and about 13%. Preferably the acid is present in an amount selected from the group consisting of from about 0.001 % to about 10%, from about 0.01% to about 5%, from about 0.05% to about 2.5% and from about 0.1% to about 1.0%.The composition can include an acceptable carrier or base.

Acceptable topical carriers are selected from traditional water and oil emulsions, suspensions, colloids, micro-emulsions, clear solutions, suspensions of nanoparticles, emulsions of nanoparticles, or anhydrous compositions. A preferred topical carrier is a cream or a powder.

The present invention also discloses a method of treating a skin condition comprising topically applying to the skin, for a period of time and in an amount sufficient to effect changes in the dermis, the composition(s) listed above. The composition(s) may be applied to an individual's skin within a region in which prevention and/or treatment is desired. The composition(s) may be applied on an as-needed basis or periodically over an extended period of time, such as, e.g., at every diaper change, at least once a day or at least once a week. It is preferred to apply the composition(s) of the present invention as frequently as possible.

The pH of the composition may be about 4.5 to about 8. Preferably, the pH of the composition is about 5 to about 6. More preferably, the pH of the composition is about 5 to about 5.5. Most preferably, the pH of the composition is about 5.5.

### EXAMPLES

The following examples are presented to illustrate the invention. The scope of the invention is defined by the appended claims. All quantities are in weight percent. As used herein, the unit "U" refers to "enzyme unit", known in the art. One "U" is defined as the amount of the enzyme that catalyzes the conversion of 1 micro mole of substrate per minute.

### Example 1

### The effect of cations on trypsin inhibition.

The objective of the following experiment was to determine whether and to what extent the following cations inhibited trypsin activity in an in vitro assay.

The inhibition of trypsin-induced cleavage of a fluorescent casein peptide was measured using the EnzChek™ protease assay kit, following manufacturer's instructions (EnzChek™ Protease Assay Kits Product Information, Revised; Molecular Probes, Eugene OR). Test materials at different concentrations (as outlined in Table 1) were prepared in 1x phosphate buffered saline (PBS, pH 7.4). Trypsin working solution (Sigma, St. Louis, MO, unit/mL) was prepared in a portion of the digestion buffer provided in the assay kit (the digestion buffer provided in the assay kit comprises, in total, 260mL of 200nM Tris-HCl, pH7.8, containing 40mM sodium azide). Stock solution of BODIPY FL casein (the trypsin substrate, comprising a casein derivative labeled with a pH-insensitive green fluorescent protein, mg/mL) was prepared by adding 0.2 mL of phosphate buffered saline (PBS) to the substrate vials provided in kit, allowing sufficient time at room temperature for the substrate to dissolve fully (each substrate vial provided in the kit contains 200µg of BODIPY FL casein lyophilized from phosphate-buffered saline (PBS)). The final substrate working solution (10 microgram/mL) was prepared by dilution of 0.2 mL of the stock solution of BODIPY FL with 19.8 mL of digestion buffer (pH 7.8). To detect enzyme activity, 100µL of the BODIPY FL casein working solution was added to 50µL of trypsin working solution (containing 100U of trypsin) and 50µl of the solution of the required test material (if required) . Following incubation of trypsin, with or without the test materials, with the BODIPY fluorescent casein substrate, at room temperature for one hour, fluorescence was measured (excitation 485 nm /emission 530 nm) using a SpectraMax microtiter plate reader (Molecular Devices Corporation, Sunnyvale, CA) and Softmax Pro software (Molecular Devices Corporation). Each experiment was performed in three replicates. The results of the experiment are shown in Table 1. The percentage activity is quoted with comparison to results obtained from the assay performed without the addition of a test substrate. In general, the results demonstrate that divalent cations are more potent than monovalent cations in their trypsin inhibitory activity.

**Table 1**

| Chemical name | Molecular formula | Molecular weight | Concentrati on % (g/mL) | Concentrati on (mM) | % trypsin inhibitory activity |
|---|---|---|---|---|---|
| (II) chloride dihydrate | CuCl₂·(H₂O)₂ | 170.48 | 0.01% | 0.59 | 64.98 |
| | | | 0.10% | 5.87 | 99.34 |
| | | | 1% | 58.66 | 108.34 |
| Zinc chloride | ZnCl₂ | 136.3 | 0.01% | 0.73 | 42.99 |
| | | | 0.10% | 7.34 | 88.51 |
| | | | 1% | 73.37 | 105.87 |
| Iron (II) chloride tetrahydrate | FeCl₂·(H₂O)₄ | 198.81 | 0.01% | 0.50 | -3.24 |
| | | | 0.10% | 5.03 | 64.84 |
| | | | 1% | 50.3 | 112.23 |
| Calcium chloride | CaCl₂ | 110.98 | 0.01% | 0.9 | -10.63 |
| | | | 0.10% | 9.01 | 46.52 |
| | | | 1% | 90.11 | 71.35 |
| Strontium chloride | SrCl₂ | 158.53 | 0.01% | 0.63 | 6.51 |
| | | | 0.10% | 6.31 | 11.78 |
| | | | 1% | 63.08 | 47.05 |
| Magnesium chloride | MgCl₂ | 95.21 | 0.01% | 1.05 | 3.29 |
| | | | 0.10% | 10.5 | 12.72 |
| | | | 1% | 105.03 | 39.32 |
| Sodium chloride | NaCl | 58.44 | 0.01% | 1.71 | -7.57 |
| | | | 0.10% | 17.11 | -4.15 |
| | | | 1% | 171.12 | 10.88 |
| Lithium chloride | LiCl | 42.39 | 0.01% | 2.36 | 8.71 |
| | | | 0.10% | 23.59 | 7.56 |
| | | | 1% | 235.9 | -0.56 |
| Silver nitrate | AgNO₃ | 169.87 | 0.01% | 0.59 | -2.43 |
| | | | 0.10% | 5.89 | 2.91 |
| | | | 1% | 58.87 | 5.37 |

The results demonstrate that, overall, divalent cations are more potent than monovalent cations in trypsin inhibitory activity. In particular, the results of Example 1 demonstrate that with regard to trypsin inhibition, activity is in the following order: Zn⁺² > Fe⁺² > Ca⁺² > Sr⁺² >Mg⁺². The results also show that, overall, all of the divalent cations have higher activity than the monovalent cations, Na⁺¹, Ag⁺¹, and Li⁺¹.

### Example 2: The effect of salt form of the same divalent cation on trypsin inhibition.

The objective of the following experiment was to determine whether and to what extent different salt forms of the same divalent cation had on inhibition of trypsin activity in an in vitro assay.

Trypsin inhibitory activity was analyzed as described in Example 1 and the effect of different salt forms of the same cations on the trypsin inhibitory activities was assessed. Table 2 shows the molecular weights, concentration and trypsin inhibitory activity of tested compounds.

**Table 2**

| Chemical name | Molecular formula | Molecular weight | % weight(g/mL) | mM | % trypsin inhibitory activity |
|---|---|---|---|---|---|
| Zinc chloride | ZnCl₂ | 136.32 | 0.01 % | 0.73 | 41.25 |
| | | | 0.10% | 7.34 | 87.69 |
| | | | 1% | 73.36 | 98.75 |
| Zinc acetate dihydrate | Zn( C₂H₃O₂)₂·(H₂O)₂ | 219.5 | 0.01% | 0.46 | 36.22 |
| | | | 0.10% | 4.56 | 68.92 |
| | | | 1% | 45.56 | 93.8 |
| Zinc gluconate | Zn(C₆H₁₁O₇)₂ | 455.68 | 0.01% | 0.22 | 25.33 |
| | | | 0.10% | 2.19 | 40.93 |
| | | | 1% | 21.94 | 85.52 |
| Zinc lactate | Zn(C₆H₁₁O₇)₂ | 243.55 | 0.01% | 0.41 | 23.01 |
| | | | 0.10% | 4.11 | 36.36 |
| | | | 1% | 41.06 | 87.95 |
| Copper (II) acetate monohydrate | Cu(C₂H₃O₂)₂·(H₂O) | 199.65 | 0.01% | 0.50 | 66.53 |
| | | | 0.10% | 5.01 | 95.17 |
| Copper (II) gluconate | Cu(C₁₂H₂₂O₁₄) | 453.8 | 0.01% | 6.32 | 14.38 |
| | | | 0.10% | 63.22 | 65.52 |
| Calcium chloride | CaCl₂ | 110.98 | 0.10% | 9.01 | 33.41 |
| | | | 1% | 90.11 | 71.39 |
| Calcium acetate monohydrate | Ca( C₂H₃O₂)₂·(H₂O) | 176.17 | 0.10% | 5.68 | 14.38 |
| | | | 1% | 56.76 | 65.52 |
| Magnesium chloride | MgCl₂ | 95.21 | 0.10% | 10.53 | 9.02 |
| Magnesium acetate tetrahydrate | Mg( C₂H₃O₂)₂·(H₂O)₄ | 214.45 | 0.10% | 4.66 | -3.09 |

The results demonstrate that different salt forms of similar cations have different potencies in terms of trypsin inhibitory activity.

As shown in Table 2, Zn²⁺ in the chloride salt form has the highest trypsin inhibition activity, followed by the acetate, gluconate and lactate salt forms when calibrated by molar concentrations. Table 2 also shows that, for a given divalent cation, a similar ordering of anions in trypsin inhibitory activities is observed. Therefore, choosing the anion could affect the trypsin inhibitory activity of the divalent cation-containing composition.

### Example 3

The objective of the following experiment was to determine whether and to what extent adding a divalent cation to talc inhibited trypsin activity in an in vitro assay. The inhibition of trypsin-induced cleavage of a fluorescent casein peptide was measured using the EnzChek™ protease assay kit, following manufacturer's instructions (EnzChek™ Protease Assay Kits Product Information, Revised; Molecular Probes, Eugene OR). Mixtures of talc stock solutions with (1) divalent cation salts at different concentrations (as indicated in Table 3) and (2) magnesium chloride at different concentrations (as indicated in Table 4) were prepared in lx phosphate buffered saline (PBS, pH 7.4). Trypsin working solution (Sigma, St. Louis, MO, unit/mL) was prepared in a portion of the digestion buffer provided in the assay kit (the digestion buffer provided in the assay kit comprises, in total, 260mL of 200nM Tris-HCl, pH7.8, containing 40mM sodium azide). Stock solution of BODIPY FL casein (the trypsin substrate, comprising a casein derivative labeled with a pH-insensitive green fluorescent protein, mg/mL) was prepared by adding 0.2 mL of phosphate buffered saline (PBS) to the substrate vials provided in kit, allowing sufficient time at room temperature for the substrate to dissolve fully (each substrate vial provided in the kit contains 200µg of BODIPY FL casein lyophilized from phosphate-buffered saline (PBS)). The final substrate working solution (10 microgram/mL) was prepared by dilution of 0.2 mL of the stock solution of BODIPY FL with 19.8 mL of digestion buffer (pH 7.8). To detect enzyme activity, 100µL of the BODIPY FL casein working solution was added to 50µL of trypsin working solution (containing 100U of trypsin) and 50µl of the solution of the required test material (if required) . Following incubation of the trypsin, with or without the test materials, with the BODIPY fluorescent casein substrate, at room temperature for one hour, fluorescence was measured (excitation 485 nm /emission 530 nm) using a SpectraMax microtiter plate reader (Molecular Devices Corporation, Sunnyvale, CA) and Softmax Pro software (Molecular Devices Corporation). Each experiment was performed in three replicates. The percentage activity is quoted with comparison to results obtained from the assay performed without the addition of a test substrate. The results of the experiments are shown in Table 3 and Table 4.

**Table 3**

| Samples | Salt alone | With Talc | Hypothetically calculated additive value of salt and Talc | % synergistic activity relative to hypothetic additive value |
|---|---|---|---|---|
| | trypsin inhibition activities (%) | | | |
| Talc | | 8.55 | 8.55 | 0.00 |
| CaCl₂ | 17.95 | 26.43 | 26.50 | -0.26 |
| MgCl₂ | 4.75 | 20.05 | 13.30 | 50.75 |
| FeCl₂·(H₂O)₄ | 65.07 | 64.53 | 73.62 | -12.35 |
| Ca(C₂H₃O₂)₂·(H₂O) | 15.31 | 24.6 | 23.86 | 3.10 |
| Zn(C₂H₃O₂)₂·(H₂O)₂ | 62.84 | 60.18 | 71.39 | -15.70 |
| ZnCl₂ | 81.07 | 77.96 | 89.62 | -13.01 |
| Mg(C₂H₃O₂)₂·(H₂O)₄ | 0.83 | 17.83 | 9.38 | 90.09 |

| | | | | |
|---|---|---|---|---|
| All tested materials were at 0.1% (g/mL). *Hypothetical additive value was calculated by adding % trypsin inhibition of the salt alone and talc alone (8.55%). **% synergistic activity was calculated by subtracting the hypothetical additive value from the experimentally measured "with talc value" then dividing by the hypothetical additive value. | | | | |

The use of talc is soothing to diaper rash. The results suggest that a formulation containing magnesium divalent cation and talc could not only provide soothing relief but also inhibit the progression of the rash by inhibiting trypsin activity.

**Table 4**

| Samples | Salt alone | With 1% Talc | Hypothetically calculated additive value of salt and Talc | % synergistic activity relative to hypothetical additive value |
|---|---|---|---|---|
| | trypsin inhibition activities (%) | | | |
| no MgCl₂ | 0 | 3.42 | 3.42 | 0.00 |
| 0.1% MgCl₂ | 9.02 | 25.43 | 12.44 | 104.42 |
| 0.5% MgCl₂ | 19.51 | 32.84 | 22.93 | 43.22 |
| 1% MgCl₂ | 22.13 | 40.42 | 25.55 | 58.20 |

| | | | | |
|---|---|---|---|---|
| All concentrations listed are g/mL. | | | | |

The results demonstrate that talc and magnesium chloride alone have moderate to low levels of trypsin inhibitory activity, and that the combination of talc and magnesium chloride had a synergistic increase in trypsin inhibition.

### Example 4: Trypsin inhibition with zinc oxide containing formulation.

The objective of the following experiment was to determine whether and to what extent adding an acid to a formulation containing zinc oxide had on inhibition of trypsin activity in an in vitro assay. The inhibition of trypsin-induced cleavage of a fluorescent casein peptide was measured using the EnzChek^{TM} protease assay kit, following manufacturer's instructions (EnzChek^{TM} Protease Assay Kits Product Information, Revised; Molecular Probes, Eugene OR). Mixtures of zinc oxide and acid at different concentrations were prepared in lx PBS (pH 7.4) and their pH was recorded as listed in the Tables 5-8. Trypsin working solution (Sigma, St. Louis, MO, unit/mL) was prepared in a portion of the digestion buffer provided in the assay kit (the digestion buffer provided in the assay kit comprises, in total, 260mL of 200nM Tris-HCl, pH7.8, containing 40mM sodium azide). Stock solution of BODIPY FL casein (the trypsin substrate, comprising a casein derivative labeled with a pH-insensitive green fluorescent proten, mg/mL) was prepared by adding 0.2 mL of phosphate buffered saline (PBS) to the substrate vials provided in kit, allowing sufficient time at room temperature for the substrate to dissolve fully (each substrate vial provided in the kit contains 200µg of BODIPY FL casein lyophilized from phosphate-buffered saline (PBS)). The final substrate working solution (10 microgram/mL) was prepared by dilution of 0.2 mL of the stock solution of BODIPY FL with 19.8 mL of digestion buffer (pH 7.8). To detect enzyme activity, 100µL of the BODIPY FL casein working solution was added to 50µL of trypsin working solution (containing 100U of trypsin) and 50 µl of the solution of the required test material (if required). Following incubation of the trypsin, with or without the test materials (50 µl), with the BODIPY fluorescent casein substrate, at room temperature for one hour, fluorescence was measured (excitation 485 nm /emission 530 nm) using a SpectraMax microtiter plate reader (Molecular Devices Corporation, Sunnyvale, CA) and Softmax Pro software (Molecular Devices Corporation). Each experiment was performed in three replicates.

For preparations, samples were diluted in lx PBS at 1:10 ratio and subjected to centrifugation at 13,200 rpm for 1 min using Eppendorf Centrifuge (Model 5415D, Eppendorf AG, Hamburg, Germany) to remove the solid formulation ingredients. The supernatants (50 µl) were used for trypsin inhibitory activity analyses as described above. For preparations, samples were diluted in 10, or 20 or 40 % ethanol and subjected to centrifugation at 13,200 rpm for 1 min using Eppendorf Centrifuge (Model 5415D, Eppendorf AG, Hamburg, Germany) to remove the solid formulation ingredients. The supernatants (50 µl) were used for trypsin inhibition assay. Possible effects of ethanol solution (10, 20 or 40% ethanol) on trypsin inhibition activity were tested in the same study and found no significant impact. The percentage activity is quoted with comparison to results obtained from the assay performed without the addition of a test substrate. The results of the experiments are shown in Tables 5, 6, 7 and 8.

### Preparations 1 and 2. Zinc oxide containing formulation without or with varying concentrations of acetic acid.

Two preparations of the same formulation were prepared on different days. The base formulation for each preparation contained the ingredients listed below and was prepared as follows: 1. Prepare 1 N acetic acid solution. 2. In a beaker weigh the amount of water needed for each formulation and begin heating to 65-70 °C. 3. Add methyl and propyl paraben at 70 °C and let it stir for 5 minutes and reduce the heat. 4. Add the appropriate amount of acetic acid (control contains no acetic acid). 5. Add zinc oxide and let it react for 5 minutes at 55-60 °C. 6. Add mineral oil and petrolatum to the mixture and after the petrolatum melts turn the heat off and let the mixture cool with constant stirring.7. Measure the pH.

The trypsin inhibitory activity for the two preparations are shown in Table 6. The preparations exhibited instability over time.

| **Preparations 1 and 2** |
|---|
| 10 % zinc oxide |
| acetic acid at varying concentrations (no acid; 0.1-0.6%) |
| 0.18 % methyl paraben |
| 0.02% propyl paraben |
| 19.39 % mineral oil |
| 25.86 % petrolatum |
| water at varying concentrations (32.34-34%). |

### Preparations 3, 4 and 5. Trypsin inhibitory activity of formulation containing (1) zinc oxide and no citric acid; (2) zinc oxide and 0.1% citric acid; (3) zinc oxide and 0.5% citric acid.

Preparations 3, 4, and 5, the results for which are presented in Tables 7 and 8 below, contained the ingredients listed below and were prepared as follows:

### Preparation 3

| | Ingredient | %wt/wt | for 200 g |
|---|---|---|---|
| Oil phase | C12-15alkylbenzoate | 5.53 | 11.05 |
| | Isopropyl Palmitate | 4.42 | 8.84 |
| | Zinc Stearate | 0.50 | 1.00 |
| | Dicaprylyl carbonate | 5.53 | 11.05 |
| | Polyglyceryl-2 dipolyhydroxystearate | 3.32 | 6.64 |
| | Polyglyceryl-3 diisostearate | 2.21 | 4.42 |
| | Lanolin (99.98%)/butylhydroxytoluene (0.02%) ("lanolin BHT") | 4.00 | 8.00 |
| | Zinc Oxide | 13.00 | 26.00 |
| Water phase | Water, Demineralized | 59.43 | 118.86 |
| | Disodium EDTA | 0.20 | 0.40 |
| | Magnesium Sulfate Heptahydrate | 1.00 | 2.00 |
| | Phenoxyethanol | 0.50 | 1.00 |
| | Methylparaben | 0.30 | 0.60 |
| | Propylparaben | 0.07 | 0.14 |
| | | 100.00 | 200.00 |

### Oil Phase

1. Combine C12-15alkylbenzoate and Isopropyl Palmitate in an appropriately sized beaker and bring to 120-130 °C. Mix at 100-300 rpm until homogeneous.
2. Add Zinc Stearate at 120-130 °C. Mix at 100-300 rpm until homogeneous.
3. Allow mixture to cool to 95-105 °C. Add Dicaprylyl carbonate and mix at 100-300 rpm until homogeneous.
4. Add Polyglyceryl-2 dipolyhydroxystearate to the mixture at 90-105 °C. Mix at 100-300 rpm until homogeneous.
5. Allow mixture to cool to 85-95°C and add Polyglyceryl-3 diisostearate. Mix at 100-300 rpm until homogeneous.
6. Add lanolin BHT at 85-95 °C. Mix at 200-500 rpm until homogeneous.
7. Add Zinc Oxide at 85-95 °C. Mix at 200-500 rpm until homogeneous.
8. Homogenize mixture at 5000-6000 rpm for 5 minutes.
9. Remove mixture from homogenizer and keep at 85-90 °C

### Water Phase

10. In a separate beaker, combine Water and Disodium EDTA at 40-50 °C. Mix at 100-300 rpm until homogeneous.
11. Bring mixture to 50-60°C and add Magnesium Sulfate Heptahydrate. Mix at 100-300 rpm until homogeneous.
12. Add Phenoxyethanol at 50-60 °C. Mix at 100-300 rpm until homogeneous.
13. Add Methylparaben at 50-60 °C. Mix at 100-300 rpm until homogeneous.
14. Add Propylparaben at 50-60 °C. Mix at 100-300 rpm until homogeneous.
15. Bring mixture to 85-90°C.

### Emulsion Phase

16. Slowly add Water Phase contents into Oil Phase at 85-90 °C. Increase mix speed as necessary. Mix for 20 minutes.
17. Homogenize mixture at 85-90 °C at 6000-8000 rpm for 5 min.
18. Remove mixture from homogenizer and mix with propeller blades at mix speed used in Step 16 (+/- 20%). Remove heat and allow to cool at room temperature.
19. When mixture cools to 30-40 °C, homogenize at 6000-8000 rpm for 2 min.

### Preparation 4

| | Ingredient | %wt/wt | for 200 g |
|---|---|---|---|
| Oil phase | C12-15alkylbenzoate | 5.53 | 11.05 |
| | Isopropyl Palmitate | 4.42 | 8.84 |
| | Zinc Stearate | 0.50 | 1.00 |
| | Dicaprylyl carbonate | 5.53 | 11.05 |
| | Polyglyceryl-2 dipolyhydroxystearate | 3.32 | 6.64 |
| | Polyglyceryl-3 diisostearate | 2.21 | 4.42 |
| | Lanolin (99.98%)/butylhydroxytoluene (0.02%) | 4.00 | 8.00 |
| | Zinc Oxide | 13.00 | 26.00 |
| Water phase | Water 1, Demineralized | 39.33 | 78.66 |
| | Water 2, Demineralized | 20.00 | 40.00 |
| | Citric acid | 0.10 | 0.20 |
| | Disodium EDTA | 0.20 | 0.40 |
| | Magnesium Sulfate Heptahydrate | 1.00 | 2.00 |
| | Phenoxyethanol | 0.50 | 1.00 |
| | Methylparaben | 0.30 | 0.60 |
| | Propylparaben | 0.07 | 0.14 |
| | | 100.00 | 200.00 |

### Oil Phase

1. Combine C12-l5alkylbenzoate and Isopropyl Palmitate in an appropriately sized beaker and bring to 120-130 °C. Mix at 100-300 rpm until homogeneous.
2. Add Zinc Stearate at 120-130 °C. Mix at 100-300 rpm until homogeneous.
3. Allow mixture to cool to 95-105 °C. Add Dicaprylyl carbonate and mix at 100-300 rpm until homogeneous.
4. Add Polyglyceryl-2 dipolyhydroxystearate to the mixture at 90-105 °C. Mix at 100-300 rpm until homogeneous.
5. Allow mixture to cool to 85-95°C and add Polyglyceryl-3 diisostearate. Mix at 100-300 rpm until homogeneous.
6. Add lanolin BHT at 85-95 °C. Mix at 200-500 rpm until homogeneous.
7. Add Zinc Oxide at 85-95 °C. Mix at 200-500 rpm until homogeneous.
8. Homogenize mixture at 5000-6000 rpm for 5 minutes.
9. Remove mixture from homogenizer and keep at 85-90 °C

### Water Phase

10. In a second beaker, combine Water 1 and Citric Acid at 40-50 °C. Mix at 100-300 rpm until homogeneous.
11. In a third beaker, combine Water 2 and Disodium EDTA at 40-50 °C. Mix at 100-300 rpm until homogeneous.
12. Bring mixture to 50-60°C and add Magnesium Sulfate Heptahydrate. Mix at 100-300 rpm until homogeneous.
13. Add Phenoxyethanol at 50-60 °C. Mix at 100-300 rpm until homogeneous.
14. Add Methylparaben at 50-60 °C. Mix at 100-300 rpm until homogeneous.
15. Add Propylparaben at 50-60 °C. Mix at 100-300 rpm until homogeneous.
16. Add contents of second beaker (Water and Citric Acid solution) into third beaker and bring mixture to 85-90°C.

### Emulsion Phase

17. Slowly add Water Phase contents into Oil Phase at 85-90 °C. Increase mix speed as necessary. Mix for 20 minutes.
18. Homogenize mixture at 85-90 °C at 6000-8000 rpm for 5 min.
19. Remove mixture from homogenizer and mix with propeller blades at mix speed used in Step 17 (+/- 20%). Remove heat and allow to cool at room temperature.
20. When mixture cools to 30-40 °C, homogenize at 6000-8000 rpm for 2 min.

### Preparation 5

| | Ingredient | %wt/wt | for 200 g |
|---|---|---|---|
| Oil phase | C12-15alkylbenzoate | 5.53 | 11.05 |
| | Isopropyl Palmitate | 4.42 | 8.84 |
| | Zinc Stearate | 0.50 | 1.00 |
| | Dicaprylyl carbonate | 5.53 | 11.05 |
| | Polyglyceryl-2 dipolyhydroxystearate | 3.32 | 6.64 |
| | Polyglyceryl-3 diisostearate | 2.21 | 4.42 |
| | Lanolin (99.98 %)/butylhydroxytoluene (0.02%) | 4.00 | 8.00 |
| | Zinc Oxide | 13.00 | 26.00 |
| Water phase | Water 1, Demineralized | 38.93 | 77.86 |
| | Water 2, Demineralized | 20.00 | 40.00 |
| | Citric acid | 0.50 | 1.00 |
| | Disodium EDTA | 0.20 | 0.40 |
| | Magnesium Sulfate Heptahydrate | 1.00 | 2.00 |
| | Phenoxyethanol | 0.50 | 1.00 |
| | Methylparaben | 0.30 | 0.60 |
| | Propylparaben | 0.07 | 0.14 |
| | | 100.00 | 200.00 |

### Oil Phase

1.Combine C12-15alkylbenzoate and Isopropyl Palmitate in an appropriately sized beaker and bring to 120-130 °C. Mix at 100-300 rpm until homogeneous.
2.Add Zinc Stearate at 120-130 °C. Mix at 100-300 rpm until homogeneous.
3.Allow mixture to cool to 95-105 °C. Add Dicaprylyl carbonate and mix at 100-300 rpm until homogeneous.
4.Add Polyglyceryl-2 dipolyhydroxystearate to the mixture at 90-105 °C. Mix at 100-300 rpm until homogeneous.
5.Allow mixture to cool to 85-95C and add Polyglyceryl-3 diisostearate. Mix at 100-300 rpm until homogeneous.
6.Add lanolin BHT at 85-95 °C. Mix at 200-500 rpm until homogeneous.
7.Add Zinc Oxide at 85-95 °C. Mix at 200-500 rpm until homogeneous.
8.Homogenize mixture at 5000-6000 rpm for 5 minutes.
9.Remove mixture from homogenizer and keep at 85-90 °C

### Water Phase

10. In a second beaker, combine Water 1 and Citric Acid at 40-50 °C. Mix at 100-300 rpm until homogeneous.
11. In a third beaker, combine Water 2 and Disodium EDTA at 40-50 °C. Mix at 100-300 rpm until homogeneous.
12. Bring mixture to 50-60°C and add Magnesium Sulfate Heptahydrate. Mix at 100-300 rpm until homogeneous.
13. Add Phenoxyethanol at 50-60 °C. Mix at 100-300 rpm until homogeneous.
14. Add Methylparaben at 50-60 °C. Mix at 100-300 rpm until homogeneous.
15. Add Propylparaben at 50-60 °C. Mix at 100-300 rpm until homogeneous.
16. Add contents of second beaker (Water and Citric Acid solution) into third beaker and bring mixture to 85-90°C.

### Emulsion Phase

17. Slowly add Water Phase contents into Oil Phase at 85-90 °C. Increase mix speed as necessary. Mix for 20 minutes.
18. Homogenize mixture at 85-90 °C at 6000-8000 rpm for 5 min.
19. Remove mixture from homogenizer and mix with propeller blades at mix speed used in Step 17 (+/- 20%). Remove heat and allow to cool at room temperature.
20. When mixture cools to 30-40 °C, homogenize at 6000-8000 rpm for 2 min.

### RESULTS

**Table 5 Trypsin inhibitory activity of mixtures of zinc oxide and acetic acid.**

| ZnO concentration (%) | No acetic acid | | 0.01 % acetic acid | | 0.05% acetic acid | |
|---|---|---|---|---|---|---|
| | pH | % Trypsin inhibitory activity | pH | % Trypsin inhibitory activity | pH | % Trypsin inhibitory activity |
| No ZnO | 7.49 | 0 | 6.84 | -11.18+/-2.61 | 4.75 | 25.81+/-2.13 |
| 0.1% ZnO | 7.61 | -2.6+/-2.76 | 7.28 | 1.85+/-1.34 | 6.03 | 49.16+/-1.43 |
| 1% ZnO | 8.07 | 5.40 +/- 1.24 | 7.85 | 15.82+/-1.68 | 6.08 | 58.19+/-0.73 |

The results in Table 5 demonstrate that zinc oxide has minimal protease activity, while the mixtures of zinc oxide and acetic acid have trypsin inhibitory activity, which is dose-dependent. This suggests that the combination of zinc oxide, a known agent used to relieve diaper rash, with low concentrations of acetic acid, could have a synergistic effect by actively inhibiting the progression of the rash.

**Table 6 Trypsin inhibitory activity of formulations (preparations 1 and 2 as defined above) containing zinc oxide and acetic acid (tested at a 1:10 dilution):**

| | Preparation-1 | Preparation-2 |
|---|---|---|
| Ingredients | % trypsin inhibition | % trypsin inhibition |
| ZnO, no acetic acid | 24.35 +/- 1.34 | 23.68 +/- 2.43 |
| ZnO, 0.1 % acetic acid | 29.43 +/- 1.47 | 18.68 +/- 3.62 |
| ZnO, 0.2% acetic acid | 70.87 +/- 0.92 | 32.70 +/- 6.09 |
| ZnO, 0.3% acetic acid | | 28.53 +/- 3.12 |
| ZnO, 0.4% acetic acid | | 39.40 +/- 9.09 |
| ZnO, 0.5% acetic acid | | 24.40 +/- 4.68 |
| ZnO, 0.6% acetic acid | 50.14 +/- 4.25 | 39.13 +/- 13.75 |

The results in Table 6 suggest that a portion of the zinc oxide is converted to zinc ions through the addition of a small amount of acid into the formula, and that it is the zinc ions that are active in inhibiting trypsin activity

**Table 7. Trypsin inhibitory activity of formulation containing (1) zinc oxide and no citric acid (preparation 3); (2) zinc oxide and 0.1 % citric acid (preparation 4); (3) zinc oxide and 0.5% citric acid (preparation 5).**

| Formulation | Preparation 3 | Preparation 4 | Preparation 5 | Currently available product (Baby Paste 5.0*) |
|---|---|---|---|---|
| Ingredients | Baby diaper rash cream base with 13% ZnO | Baby diaper rash cream base with 13% ZnO and 0.1% citric acid | Baby diaper rash cream base with 13% ZnO and 0.5% citric acid | Formulation contains 25% ZnO and an unknown amount of acetic acid, pH 5 |
| % trypsin inhibitory activity (tested at 1:5 dilution) | 20.02 +/- 6.71 | 50.72 +/- 16.74 | 86.09 +/- 3.64 | 29.06 +/- 2.34 |

| | | | | |
|---|---|---|---|---|
| *Mendelson Pharmaceuticals LLC, Mountainside, NJContains 25% zinc oxide; white petrolatum; and acetic acid from vinegar. | | | | |

In a separate study, the addition of 0.5% citric acid (preparation-6) or 0.5% ZnCl₂ (preparation-7) to the pre-made baby diaper rash cream base by simple mixing was tested and compared with a commercially-available product for diaper rash after adding 0.5% citric acid (preparation-8) and simple mixing. The results are shown in Table 8.

**Table 8.**

| Formulation | Prepapration-3 | Preparation-4 | Preparation-5 | Currently available product (Baby Paste 5.0*) | Preparation-6 | Preparation-7 | Preparation-8 |
|---|---|---|---|---|---|---|---|
| Ingredients | baby diaper rash cream base (13% ZnO) | baby diaper rash cream base with 13% ZnO and 0.1% citric acid | baby diaper rash cream base with 13% ZnO and 0.5% citric acid | formulation contains 25%ZnO and an unknown amount of acetic acid, pH 5 | Adding 0.5% citric acid to pre-made baby diaper rash cream base (preparation 3, 13% ZnO) | Adding 0.5% ZnCl₂ to pre-made baby diaper rash cream base (preparation 3, 13% ZnO) | Adding 0.5% citric acid to Baby paste 5.0 |
| % trypsin inhibitory activity (tested at 1:5 dilution) | 20.02 +/- 6.71 | 6.7150.72 +/-16.74 | 86.09 +/-3.64 | 29.06 +/- 2.34 | 82.84 +/- 0.79 | 88.59 +/- 0.67 | 30.37 +/- 2.93 |

| | | | | | | | |
|---|---|---|---|---|---|---|---|
| *Mendelson Pharmaceuticals LLC, Mountainside, NJContains 25% zinc oxide; white petrolatum; and acetic acid from vinegar. | | | | | | | |

For the above study, the results are an average of three independent experiments, wherein the samples, which were diluted at 1:10 ratio in lx PBS solutions containing 10%, 20% or 40% ethanol, were tested in triplicates. The results are from an average of all conditions at the same dilution.

For the currently available product (i.e., Baby Paste 5.0, Mendelson Pharmaceuticals LLC, Mountainside, NJ), the results are an average of a single study: sample diluted at 1:10 ratio, in lx PBS solutions containing 10%, 20% or 40% ethanol, was tested in triplicate. The results are from an average of all conditions at the same dilution. The Baby Paste 5.0 (containing 25% ZnO) and baby diaper rash cream (preparation-3, containing 13% ZnO) have placebo effect in terms of low trypsin inhibitory activity (29.06% and 20.02%, respectively). This effect is most likely due to the interference of soluble or insoluble formulation ingredients (e.g., ZnO) with the assay, and therefore, it is believed that it is the result of assay conditions and not of inhibitory activity. Preparation-4 and 5 with 0.1% and 0.5% acetic acid showed acid concentration-dependent increase in trypsin inhibitory activity (50.72% and 86.09%, respectively). The placebos of these preparations, which did not contain the acetic acid, also had low trypsin inhibitory activity because of interference. In addition, preparation 1 (10% ZnO and 0.2% acetic acid), which contains similar ingredients as Baby Paste 5.0, showed significantly higher activity (71%, Table 6) than the latter (29%, Table 7). Therefore, we conclude that the Baby Paste 5.0 and the baby diaper rash cream base (preparation-1) have no significant trypsin inhibitory activity, and that it is the addition of citric acid to the baby diaper rash cream base which leads to the release of zinc ion and results in increased trypsin inhibitory activity.

The foregoing examples are not intended to limit the scope of the present invention, which is set out in the following claims. In particular, various equivalents and substitutions will be recognized by those skilled in the art in view of the foregoing disclosure and these are contemplated to be within the scope of the invention.

## Claims

1. A composition comprising a divalent cation and a carrier for use in a method of treating and/or preventing enzymatic irritation.

2. The composition for use as claimed in claim 1, where the enzymatic irritation is enzymatic dermatitis.

3. The composition for use as claimed in claim 1 or claim 2, wherein said divalent cation is selected from the group consisting of, copper (II), zinc, iron (II), calcium, strontium, magnesium, tin (II), nickel (II), manganese (II) and cadmium (II).

4. The composition for use as claimed in any preceding claim comprising a divalent cation/anion pair.

5. The composition for use as claimed in any preceding claim, wherein the enzymatic irritation is enzymatic dermatitis, and said composition comprises a divalent cation/anion pair.

6. The composition for use as claimed in claim 4 or claim 5, wherein said divalent cation/anion pair is selected from the group consisting of copper (II) chloride, copper (II) acetate, copper (II) gluconate, copper (II) lactate, zinc chloride, zinc acetate, zinc gluconate, zinc lactate, zinc carbonate, zinc borate, zinc aspartate, zinc pyrrolidone carboxylic acid, zinc citrate, iron (II) chloride, iron (II) acetate, iron (II) gluconate, iron (II) lactate, calcium chloride, calcium acetate, calcium gluconate, calcium lactate, strontium chloride, strontium acetate, strontium gluconate, strontium lactate, magnesium chloride, magnesium acetate, magnesium gluconate and magnesium lactate.

7. The composition for use as claimed in any preceding claim, wherein the divalent cation is present in an amount selected from the group consisting of from about 0.001 % to about 50%, from about 0.001% to about 30%, from about 0.01% to about 10%, from about 0.1 % to about 5.0% and from about 0.1 % to about 1.0%.

8. The composition for use as claimed in claim 7, wherein the divalent cation is present in an amount of from about 0.001% to about 30%, preferably in an amount of from about 0.01 % to about 10%, more preferably in an amount of from about 0.1% to about 5.0%, more preferably in an amount of from about 0.1% to about 1.0%.

9. The composition for use as claimed in any preceding claim, wherein the carrier is selected from the group consisting of lotion, cream, gel, stick, spray, ointment, cleansing liquid wash, solid bar, paste, powder, mousse, wipe, patch, wound dressing, adhesive bandage, hydrogel, film and diaper.

10. The composition for use as claimed in any preceding claim, wherein said composition has a pH of from about 4.5 to about 8, preferably from about 5 to about 6, more preferably from about 5 to about 5.5.

11. The composition for use as claimed in claim 10, wherein said composition has a pH of about 5.5.

12. The composition for use as claimed in any preceding claim, further comprising an agent selected from the group consisting ofketoconazole, miconazole, elubiol, allantoin, calamine, dimethicone, kaolin, petrolatum, white petrolatum, cod liver oil, lanolin, mineral oil and topical starch.

13. The composition for use as claimed in any preceding claim, wherein the enzyme is a protease.

14. The composition for use as claimed in claim 13, wherein the protease is trypsin.

15. The composition for use as claimed in claim 14, wherein the divalent cation exhibits a trypsin inhibitory activity selected from the group consisting of at least about 15%, at least about 25%, at least about 50%, at least about 60%, at least about 70%, at least about 80% and at least about 90%.
